# EUROPEAN PATENT SPECIFICATION

(11) **EP 0 614 347 B1**
(45) Date of publication and mention of the grant of the patent: **16.07.1997**
(21) Application number: 92925338.3
(22) Date of filing: 25.11.1992
(51) Int. Cl.: A61F 2/32, A61L 27/00, C08L 23/06

(54) **ULTRAHIGH MOLECULAR WEIGHT LINEAR POLYETHYLENE AND PROCESSES OF MANUFACTURE**
ULTRAHOCHMOLEKULARES LINEARES POLYAETHYLEN UND VERFAHREN ZUR HERSTELLUNG
POLYETHYLENE LINEAIRE DE POIDS MOLECULAIRE EXTREMEMENT ELEVE ET PROCEDES DE PRODUCTION

(30) Priority: 27.11.1991 US 800868
(43) Date of publication of application: 14.09.1994
(73) Proprietor: E.I. DU PONT DE NEMOURS AND COMPANY, Wilmington Delaware 19898 (US)
(72) Inventor: HOWARD, Edward, George, Jr., Hockessin, DE 19707 (US); CHAMPION, Allan, R., Wilmington, DE 19809 (US)
(74) Representative: Jones, Alan John
(86) International application number: US9210005
(87) International publication number: WO9310953

(56) References cited:
- EP-A- 0 290 141
- EP-A- 0 373 800
- JOURNAL OF POLYMER SCIENCE, POLYMER PHYSICS EDITION vol. 7, 1969, NEW YORK US pages 2051 - 2059 DAVIDSON & WUNDERLICH 'Extended-chain crystals. II. Crystallisation of polyethylene under elevated pressure

## Description

### 1. Field of the Invention

This invention relates to a novel ultrahigh molecular weight linear polyethylene (UHMWLPE). This novel UHMWLPE, in the form of a shaped article, exhibits in various embodiments a unique combination of properties making the material useful as a bearing surface, in general, but particularly useful as a prosthetic hip joint cup and as other prosthetic shapes for replacement of other joints of the human body.

### 2. Description of the Prior Art

In U.S. Patent. No. 3,944,536 (March 1976), Lupton et al. describe UHMWPE in the form of a fabricated article exhibiting an elastic modulus of 340,000 to 500,000 psi, a tensile impact strength of 140 to 600 ft lb/in², a density of 0.95 to 0.98 g/cc at 25°C, a crystalline melting point of 142 to 148°C (as measured by differential thermal analysis) and a unique crystalline form characterized by the absence of fold spacings of 5 - 200 nm (50-2000 Angstrom units (Å)) and the presence of crystal spacings of about 1000 nm (10,000 Å). The critical feature of the process of producing this UHMWPE is disclosed to involve inducing crystallization of the molten polymer above 150°C by rapidly increasing the applied pressure from an initial level of 0.1 to 100 MPa (1 to 1000 atmospheres) to a second level of 202 to 707 MPa (2000 to 7000 atmospheres) and then cooling rapidly while maintaining a pressure sufficient to maintain the polyethylene in the solid phase until the temperature is below the crystalline melting point of the polyethylene at atmospheric pressure.

In Kunstoffe German Plastics 77 (1987) pp. 617-622, in an article entitled "Ultrahigh Molecular Polyethylene for Replacement Joints", Eyrer et al. point out that the service life of joint replacements made of UHMWPE is limited. Analysis of the damage to over 250 explanted hip cups and tibial plateaus revealed a changed property profile which they explained by post-crystallization resulting from oxidative chain decomposition. They suggested optimizing the processing of polyethylene under higher pressure and higher temperature to increase the degree of crystallinity. The Eyrer et al. product displays a creep of above 5% at a compression of 1000 *psi* (6.9 N/mm²) for 24 hours at 37°C.

EP-A-0,373,800 (E. I. du Pont de Nemours and Company) discloses novel ultrahigh molecular weight linear polyethylene in the form of a shaped article, exhibiting a unique combination of properties making the material useful as a bearing surface, in general, but particularly useful as a prosthetic hip joint cup. A novel process for preparing this article is also disclosed. The application, however, does not disclose properties to be found in UHMWPE subjected to pressures of 200-800 MPa or at temperatures of 280-320°C or 340-355°C.

One of the most remarkable advances in the medical field in recent years is the development of prosthetic joints, particularly the load bearing hip. The crippled and sometimes bed ridden elderly can walk again. The key to this development is UHMWPE because, not only does it have the necessary impact strength, but it initiates no adverse blood reactions. But at present, these prosthetic joints are limited to the older, less active segment of the population because the polymer tends to creep under the pressure that a younger more active person might develop while involved in recreation or employment. The creep would cause the loss of the close tolerance required between the plastic socket and the polished metal ball attached to the femur. These changes in dimensions disturb the distribution of walking forces which in turn accelerates more creep and wear. Eventually the increased pain requires a traumatic revision operation. One objective of this invention is to provide UHMWPE prosthetic joints with improved creep resistance hence removing some of the age restriction existing on the present polyethylene joints. This invention can also function in other UHMWPE-based prosthetic devices, for example, non-conforming joint assemblies such as knees which require a special balance of properties, especially with respect to tensile modulus and creep resistance.

### DESCRIPTION OF THE INVENTION

An object of this invention is to provide tough ultrahigh molecular weight linear polyethylene (UHMWLPE) compositions and shaped articles therefrom which exhibit unusually low creep and have excellent tensile flexural properties.

UHMWLPE compositions of this invention have molecular weights of at least 400,000, preferably at least 1,000,000. Those UHMWLPE of this invention that are prepared by processing at elevated pressure also have two crystalline melting points, the higher of which is greater than 144 degrees C (as measured by differential scanning calorimetry) and the reduction in said higher melting point upon remelting being greater than 11°C, an infrared crystallinity index of at least 0.28, preferably at least 0.35, and are characterized by a crystal morphology comprising a bimodal distribution of molecular chain fold spacings, one group of said spacings being from 200 nm to 800 nm (2000 to 8000 Å) reflecting a population of very highly extended molecular chains, the other group being from 5 to 50 nm (50 to 500 Å).

According to the invention such UHMWLPE compositions exhibits a flexural modulus of 1.7-3.45 GPa (250-500 kpsi), a tensile stress at yield of 24-31 MPa (3.5-4.5 kpsi), a tensile stress at break of 28-62 MPa (4-9 kpsi), a tensile modulus of 1.7-4.8 GPa (250-700 kpsi), an elongation of 200-500%, a notched Izod impact resistance of 641-1335 Nm per m (12-25 ft.lb. per in.) of notch, a creep at a compression of 7 MPa (1 Kpsi) of less than 1.4% after 24 h at a temperature of 23°C and a relative humidity of 50%.

This product is prepared in a process consisting essentially of following steps:
(a) forming, for example by milling or casting, the article from UHMWLPE having a molecular weight of at least 400,000, preferably at least 1,000,000 and most preferably at least 6,000,000;
(b) subjecting the article to a fluid under pressure of at least 200 MPa, but not more than 269 MPa, e.g. by placing the article in a pressure vessel (e.g., an autoclave) substantially filled with a fluid that is inert to the article, preferably water, heating the article to a temperature of 190°C to 300°C preferably 200°-260°C; and maintaining the temperature and pressure for at least 0.5 hour, preferably at least one hour;
(c) thereafter, cooling at a slow rate by reducing the temperature to about 160°-170°C or below, preferably to 160°C or below, most preferably to below 140°C, while maintaining a pressure of at least 200 MPa, but not more than 269 MPa the rate preferably being at least 300 MPa, and the rate of cooling being such that temperature gradients in the shaped article are substantially avoided; and
(d) cooling to a temperature of about 130°C or below, preferably below 120°C, most preferably below 100°C, and reducing the pressure to about 100 kPa, either sequentially or simultaneously, in a manner such that remelting of the article is prevented.

Alternatively in step (b), the article, fluid, and reactor are heated to about 200-230°C, the pressure is applied and the system held at this temperature while polymer slowly loses heat of crystallization and solidifies. Also, the pressure can be applied at any temperature during the heating cycle if the temperature exceeds about 200°C. Moreover, the pressure vessel can be used more efficiently by preheating polymer outside the reactor because heating polyethylene is a very slow process due to its high heat of fusion and thermal insulating property. The polymer can be heated in an oven, preferably in an inert atmosphere to prevent oxidation, particularly when the temperature is above 160°C. Because the UHMW polyethylenes do not flow when melted, they can be handled while hot and transferred to the preheated pressure vessel without deforming.

In step (c), the polymer must be cooled slowly at the high pressure until it is fully crystallized. At 300 MPa pressure, the crystallization temperature of UHMWLPE of over 1,000,000 molecular weight is in the range of 170°C-190°C. The pressurized vessel should be cooled slowly to insure that the temperature of the polymer is not significantly above the vessel temperature, particularly if the pressure vessel construction does not permit means for measuring the temperature of the polymer itself. It is also very important in step (c), to cool in a manner that limits or avoids severe temperature gradients in the article. For example, for a 2.5 cm X 15 cm (1 inch X 6 inch) rod, a cooling rate of about 10°C per hour is usually necessary. Although cooling rates no greater than 10°C per hour are preferred, cooling rates as high as about 35°C per hour have been used to provide the product of this invention. However, these latter rates require careful control in order to limit temperature gradients during cooling. Rapid cooling will not provide the article of this invention. This invention is particularly useful for manufacturing shaped articles where temperature gradients pose a problem during the cooling step, i.e., where the article's cross-sectional dimensions are at least 2.5 cm (1 inch) x at least 2.5 cm (1 inch), usually for joints at least 2.5 cm (1 inch) x at least 5 cm (2 inches). Specifically, the importance of this step and of this invention is manifest in producing articles having as its smallest dimension 0.5 cm (0.2 inch), i.e., at least 0.5 cm (0.2 inch) in thickness. It has been found that in such articles, the temperature gradients must still be controlled by the process of this invention in order to obtain the product of this invention.

In step (d), cooling the polymer to a temperature below its melting point at any particular pressure is necessary to ensure that none of the polymer melts as the pressure is reduced, since lowering the pressure lowers the melting point.

As an optional fifth step, it is advisable to shave the surface of the article, i.e., remove approximately the outer 2 millimeters that might contain any fluid-affected polymer.

Products of the aforementioned process possess superior strength properties and resistance to creep under load, and are excellent materials for orthopedic replacement parts.

In addition to utility in the field of orthopedic replacement, the products prove useful in other applications also requiring the special properties of the products. Not only shaped articles are of interest, but also films and fibers as well as other "downstream" forms and unshaped granular forms of the product will prove useful.

According to this invention, the term, "article" includes both shaped articles and unshaped articles.

By "fluid" in the processes of this invention, is meant a liquid, vapor or gas that is inert to process conditions and does not adversely affect, or is prevented from adversely affecting, the UHMW polymer being processed. It has been found during manufacturing the product of this invention that use of a gaseous fluid can be beneficial. Specifically, in the above-described process, the shaped article formed from commercially available UHMWLPE in step (a) is placed (step (b)) in a pressure vessel containing a gaseous fluid, for example, argon. When argon is used, penetration of the gas into the polymer should be prevented by surrounding the UHMWLPE article with a thin stainless steel or similar metal container as described in U.S. Patent No. 5,037,928 (Li et al.). As previously mentioned, other gaseous fluids may be used in place of argon so long as they do not adversely affect, or are prevented from adversely affecting, the polymer and are otherwise inert under process conditions.

After placing the suitably protected polymeric article into the gas-containing pressure vessel, a pressure of at least 200 MPa, but not more than 269 MPa, is applied and the vessel is heated to about 220°C for about 6 hours. Thereafter, the temperature is "ramped" down preferably at a rate no greater than about 10°C per hour to between 160°C and 170°C while maintaining the pressure above 200 MPa but not more than 269 Pa. The temperature may then be "ramped" down at a maximum rate to 50°C while maintaining the high pressure, after which the pressure is released.

As already indicated, a very important property of the products of this invention is creep resistance. For prosthetic devices, e.g., knee, hip, elbow joints, etc., any substantial creep can be devastating in the loss of the benefits of extremely expensive surgery. For some applications, still lower creep, higher stiffness, higher elongation, and particularly higher tensile strengths at yield are necessary. It has been found that products having these properties can be obtained by inserting between steps (a) and (b) in the four-step pressurized process described herein above a preliminary step wherein the starting UHMWLPE is heated to a temperature of 280°-355°C, preferably 320°C-355°C, et atmospheric pressure in an inert atmosphere for at least 0.5 hour, preferably at least 3 hours. The polymer should be heated as near as possible to, without reaching, its decomposition temperature. The hot polymer should be cooled slowly because very rapid cooling, such as immersion in cold water, causes internal voids to form. Voids result from a combination of large volume change (about 30%) on melting and poor heat conductivity in polyethylene. It is convenient to allow the polymer to cool wrapped in insulation. The hot polyethylene can be placed directly into the hot pressure vessel, or first cooled, then reheated to the normal 200°C and placed in the pressure vessel.

The polyethylene product prepared as just described from either the folded chain product or from conventional UHMWLPE having a molecular weight of at least 400,000, exhibits a flexural modulus of 1.7-3.5 GPa (250-500 kpsi), a tensile stress at yield of 24-31 MPa (3.5-4.5 kpsi), a tensile stress at break of 28- 62 MPa (4-9 kpsi), a tensile modulus of 2.1-4.8 GPa-(300-700 kpsi), a notched Izod impact resistance of 641-1335 Nm per m (12-25 ft.lb. per inch) of notch, an elongation at break of 200-500%, a creep at compression of 7 MPa (1 kpsi) of less than 1.4% after 24 h at a temperature of 23°C and a relative humidity of 50% and an infrared crystallinity index of at least 0.28. The product is further characterised by having two crystalline melting points, the higher of which is greater than 144°C, the reduction in said melting point upon remelting being greater than 11°C, and a bimodal distribution of fold spacings, one group of said spacings being from 5 to 200 nm (50 to 2000 Angstroms), the other group being from 5 to 50 nm (50 to 500 Angstroms).

Certain preferred embodiments of this product of the invention have tensile moduli of at least 2450 MPa (350 kpsi), and a creep value of less than 0.6%.

It is envisaged that the additional preliminary step of heating the starting UHMWLPE to 280°-355°C will also provide superior characteristics to the product described by Li.

By inert atmosphere in the processes of this invention is meant a gaseous or vaporous environment that is stable and inert to process conditions. Suitable gases include nitrogen and the noble gases. Suitable vapors include those of nonflammable, chemically inert and thermally stable liquids such as the perfluoroalkylpolyethers. Vacuum may also be employed but is not preferred.

For purposes of this invention, ultrahigh molecular weight linear polyethylene (UHMWLPE) is defined as a linear polyethylene having an estimated weight-average molecular weight greater than about 400,000, usually 1,000,000 to 10,000,000 as defined by a melt index (ASTMD-1238) of essentially zero and a reduced specific viscosity (RSV) greater than 8, preferably 25-30. The relationships of RSV to intrinsic viscosity and to molecular weight are those developed by R. Chaing as presented by P. S. Francis et al. in J. Polymer Science, 31, 453 (1958).

Another characteristic property of the products of this invention is their infrared crystallinity index (IRCI). This property, which fairly accurately reflects product crystallinity, is higher than in conventional UHMW polyethylene. To determine this index, samples are first obtained by microforming thin sections. Heat should be avoided during preparation of the samples. ICRI is the ratio of the band at 1894 reciprocal centimeters (cm⁻¹) to the band at 1305 reciprocal centimeters (cm⁻¹). Since the band at 1894 cm⁻¹ is attributed to the crystalline nature of the material and the band at 1305 cm⁻¹ is attributed to its amorphous nature, ICRI increases as the crystallinity increases. The product of this invention displays an IRCI of at least about 0.28, preferably at least 0.35. In fact, values of 0.73 and higher have been obtained. On the other hand, IRCI values for prior known UHMWLPE's seldom reach above 0.3.

In the examples, most of the properties are measured using standard ASTM tests.

All of the physical measurements were carried out under constant humidity (50% relative humidity) and temperature (23°C) conditions.

Tensile modulus, ultimate tensile strength, yield strength and elongation were measured according to ASTM D-638 with the following modifications:
- samples machined into shape without lubricating fluid
- type I tensile bar
- cross head speed = 0.5 cm (0.2")/min for tensile modulus
   5.0 cm (2.0")/min for tensile stress and elongation.

Resistance to deformation (creep) was measured in accordance with ASTM D-621 with the following modifications:
- samples machined into cylinders or cubes without the use of lubricating fluids
- samples measured 1.3 cm (0.5") x 1.3 cm (0.5") x 1.3 cm (0.5")

Flexural properties were measured according to ASTM D-790 with the following modifications:
- samples machined into shape without the use of lubricating fluids
- typical flex bar measures 0.3 cm (0.125") thick x 1.3 cm (0.5") width x 12.7 cm (5") length
- span or gage is 5.0 cm (2.0"). (This was determined by a span/depth ratio of 16/1.)
- cross head speed = 0.13 cm (0.05")/min (calculated based on span).

Impact resistance was measured using the notched Izod test given in ASTM D-256 with the following modifications:
- samples machined into shape without the use of lubricating fluid
- type A or notched IZOD
- specimen size is 1.3 cm (0.5") x 6.3 cm (2.5")
- 1 cm (0.4") from bottom of vertex to opposite side
- 3.2 cm (1.25") impacted end (from end of bar to vertex of notch)
- the notch should be the specified angle of 22.5 degrees.

It should be appreciated that in all embodiments of the invention the step of forming the article by milling, casting, or the like from UHMWLPE may be performed as the first step in the process (i.e., before heating or preheating) or as the last step in the process (i.e., after the cooling step).

The present invention is illustrated by the following non-limiting examples.

### EXAMPLE 1

A reactor with a configuration similar to that shown in Figure 2 having an internal diameter of 13 cm (5") and a length of approximately 1.8 m (70") was charged with a 10.2 cm (4") x 152 cm (60") rod of Hoechst Hostalen GUR 415 UHMWPE manufactured by Poly Hi, Inc. The closed vessel was evacuated, filled with water, and heated to 250°C over a period of 1.5 h. This temperature was maintained for an additional 2 h to insure that the UHMWPE rod was heated uniformly and no significant temperature gradients were present. The pressure was then raised to 228 MPa (33,000 psi). This pressure was maintained for the duration of the experiment. After 1 h, the temperature was ramped down to 175°C over a period of 3 h (cooling rate approximately 25°C/h). The temperature of 175°C was maintained for 1 h, and the reactor was then cooled to 75°C over a period of 2 h. The pressure was then released, and the rod was removed from the autoclave.

A 30.5 cm (12") length was cut from the rod for evaluation. A sample from the center of the rod was used for DSC analysis. The melting curve showed two peaks, one at 139°C and one at 148°C, with the higher peak being the larger. When the sample was cooled and reheated, a single melting peak at 135°C was observed. Samples from the center of the rod had a density of 0.946 g/ml and a crystallinity index (IRCI) of 0.354. ASTM Type I tensile specimens were prepared form the rod, and the following test results were obtained:

| | |
|---|---|
| Modulus, MPa (kpsi) | 2270 (329) |
| Tensile Stress (yield, MPa (kpsi)) | 26.15 (3.79) |
| Tensile Stress (break, MPa (kpsi)) | 34.1 (4.94) |
| Elongation, % | 334 |

Creep specimens in the form of 1.3 cm (0.5") cubes were also prepared from the center of the rod. A creep of 1.4% was observed under 6.9 MPa (1000 psi) load.

### EXAMPLES 2-8

The procedure of Example 1 was employed in the subsequent examples, except that Examples 2, 4 and 7 the Hoechst Hostalen GUR 415 UHMWPE rod dimensions were 7.6 cm (3") x 152 cm (60"). The enhancement temperature in each example was 250°C. Enhancement pressure and properties, determined as previously described, are given in the table below.

| Ex. | Press MPa (kpsi) | Modulus MPa (kpsi) | TS(Y) MPa (kpsi) | TS(B) MPa (kpsi) | Creep (%) | IRCI | Density (g/ml) |
|---|---|---|---|---|---|---|---|
| 2 | 228 (33) | 2036 (295) | 25.5 (3.69) | 33.3 (4.82) | 1.4 | 0.333 | 0.943 |
| 3 | 200 (29) | 1932 (280) | 24.15 (3.50) | 33.3 (4.82) | | 0.304 | 0.940 |
| 4 | 200 (29) | 1760 (255) | 24.2 (3.51) | 32.3 (4.68) | | 0.281 | 0.938 |
| 5 | 241.5 (35) | 2491 (361) | 27.3 (3.96) | 38.2 (5.53) | 1.2 | 0.373 | 0.949 |
| 6 | 255 (37) | 2795 (405) | 28.4 (4.12) | 38.2 (5.53) | 0.97 | 0.401 | 0.951 |
| 7 | 255 (37) | 2512 (364) | 27.5 (3.98) | 36.6 (5.30) | | 0.410 | 0.951 |
| 8 | 269 (39) | 2926 (424) | 28.4 (4.12) | 38.4 (5.56) | 0.95 | 0.419 | 0.951 |

The products of Examples 2-8 each display two peaks in their DSC melting point curves. This bimodal characteristic indicates that Examples 2-8 are composites of the extended chain form of the higher molecular weight fraction of the polymer and of the folded chain form of the lower molecular weight fraction of the polymer. The results of Examples 2-8 further indicate that compositions may be altered by choice of polyethylene of different weight and molecular distributions.

## Claims

1. An ultra high molecular weight linear polyethylene having a molecular weight of at least 400,000, an infrared crystallinity index of at least 0.28; a flexural modulus of 250-500 kpsi (1.7-3.45 GPa); a tensile stress at yield of 3.5 to 4.5 kpsi (24-31 MPa); a tensile stress at break of 4-9 kpsi (28-62 MPa), a tensile modulus of 250-700 kpsi (1.7-4.8 GPa); an elongation at break of 200-500%; and a Notch Izod impact resistance of 12-25 foot pounds/inch (641-1335 Nm per M) of notch, creep at a compression of 1 kpsi (7 MPa) of less than 1.4% after 24 hours at a temperature of 23°C and a relative humidity of 50%; wherein the polyethylene has two crystalline melting points, the higher of which is greater than 144°C, the reduction in said higher melting point upon remelting being greater than 11°C, characterised in that the polyethylene has a crystal morphology comprising a bimodal distribution of molecular chain fold spacings, one group of said spacings being from 200 to 800 nm, the other group being from 5 to 50 nm.

2. The polyethylene of claim 1 in the form of an article wherein its dimensions are at least 2.5 cm by at least 2.5 cm.

3. The polyethylene of claim 1 in the form of an article wherein the smallest dimension is at least 0.5 cm.

4. A process for preparing the polyethylene of claim 1 consisting essentially of the steps:
(a) forming an article of an ultrahigh molecular weight linear polyethylene having a molecular weight of at least 400,000;
(b) subjecting said article to a fluid under pressure of at least 200 MPa but not more than 269 MPa and a temperature of 190°-300°C for at least 0.5 hour;
(c) reducing the temperature to about 160-175°C or lower while maintaining the pressure at at least 200 MPa but not more than 269 MPa, the rate of reduction in temperature being such that temperature gradients in the shaped article are substantially avoided; and
(d) cooling to a temperature of about 130°C or lower and reducing the pressure to about 100 kPa in a manner such that remelting of said article is prevented.

5. The process of claim 4 wherein step (a) is performed after step (d).

6. The process of claim 4 wherein said fluid is water.

7. The process of claim 4 wherein said temperature in step (b) is 200°-260°C.

8. The process of claim 4 wherein the temperature and pressure in step (d) is maintained for at least one hour.

9. The process of claim 4 wherein the surface of the article is shaved after step (d).

10. The process of claim 4 wherein the cooling rate in step (c) is no greater than 35°C per hour.

11. The process of claim 10 wherein the cooling rate in step (c) is no greater than 10°C per hour.

## Patentansprüche

1. Lineares Polyethylen mit einer ultrahohen Molmasse von wenigstens 400 000, einem Infrarot-Kristallinitätsindex von wenigstens 0,28; einem Biegemodul von 250 - 500 kpsi (1,7 - 3,45 GPa); einer Streckspannung von 3,5 bis 4,5 kpsi (24 - 31 MPa); einer Reißspannung von 4 - 9 kpsi (28 - 62 MPa); einem Zugmodul von 250 - 700 kpsi (1,7 - 4,8 GPa); einer Reißdehnung von 200 - 500 % und einer Kerbschlagzähigkeit nach Izod von 12 - 25 Foot Pounds/Inch (641 - 1335 Nm/m) Kerbe, einem Kriechen bei einer Kompression von 1 kpsi (7 MPa) von weniger als 1,4 % nach 24 h bei einer Temperatur von 23 °C und einer relativen Feuchtigkeit von 50 %; wobei das Polyethylen zwei Kristall-Schmelzpunkte aufweist, wobei der höhere höher als 144 °C ist, die Verminderung des höheren Schmelzpunkts nach dem erneuten Schmelzen größer als 11 °C ist, dadurch gekennzeichnet, daß das Polyethylen eine Kristallmorphologie aufweist, die eine bimodale Verteilung der Faltungszwischenräume der Molekülketten umfaßt, wobei eine Gruppe der Zwischenräume 200 bis 800 nm beträgt, die andere Gruppe 5 bis 50 nm beträgt.

2. Polyethylen nach Anspruch 1 in Form eines Gegenstandes, wobei dessen Abmessungen wenigstens 2,5 cm mal wenigstens 2,5 cm betragen.

3. Polyethylen nach Anspruch 1 in Form eines Gegenstandes, wobei die kleinste Abmessung wenigstens 0,5 cm beträgt.

4. Verfahren zur Herstellung des Polyethylens nach Anspruch 1, umfassend im wesentlichen die Schritte des:
(a) Formens eines Gegenstandes aus einem linearen Polyethylen mit einer ultrahohen Molmasse von wenigstens 400 000;
(b) Einwirkenlassens eines Fluids unter einem Druck von wenigstens 200 MPa, aber nicht mehr als 269 MPa, und einer Temperatur von 190 °C - 300 °C für wenigstens 0,5 h auf den Gegenstand;
(c) Verminderns der Temperatur auf etwa 160 - 175 °C oder darunter, während der Druck auf wenigstens 200 MPa, aber nicht mehr als 269 MPa gehalten wird, wobei die Geschwindigkeit des Verminderns der Temperatur so ist, daß Temperaturgradienten im geformten Gegenstand im wesentlichen vermieden werden; und
(d) Abkühlens auf eine Temperatur von etwa 130 °C oder darunter und das Vermindern des Drucks auf etwa 100 kPa auf solche Weise, daß das erneute Schmelzen des Gegenstandes verhindert wird.

5. Verfahren nach Anspruch 4, wobei Schritt (a) nach Schritt (d) durchgeführt wird.

6. Verfahren nach Anspruch 4, wobei das Fluid Wasser ist.

7. Verfahren nach Anspruch 4, wobei die Temperatur in Schritt (b) 200 °C bis 260 °C beträgt.

8. Verfahren nach Anspruch 4, wobei die Temperatur und der Druck in Schritt (d) wenigstens 1 h lang aufrecht erhalten werden.

9. Verfahren nach Anspruch 4, wobei die Oberfläche des Gegenstandes nach Schritt (d) geformt wird.

10. Verfahren nach Anspruch 4, wobei die Abkühlgeschwindigkeit in Schritt (c) nicht höher als 35 °C/h ist.

11. Verfahren nach Anspruch 10, wobei die Abkühlgeschwindigkeit in Schritt (c) nicht höher als 10 °C/h ist.

## Revendications

1. Un polyéthylène linéaire de poids moléculaire ultra-haut ayant un poids moléculaire d'au moins 400 000, un indice de cristallinité en infrarouge d'au moins 0,28, un module de flexion de 1,7 à 3,45 GPa, une contrainte de traction à la limite d'élasticité de 24 à 31 MPa, une contrainte de traction à la rupture de 28 à 62 MPa, un module de traction de 1,7 à 4,8 GPa, un allongement à la rupture de 200 à 500 % et une résistance au choc Izod sur barreau entaillé de 641 à 1335 Nm par mètre d'entaille, un fluage sous compression de 7 MPa de moins de 1,4 % après 24 heures à une température de 23 °C et une humidité relative de 50 %, dans lequel le polyéthylène présente deux points de fusion cristalline dont le plus élevé est supérieur à 144 °C, la diminution dudit point de fusion supérieur sous l'effet d'une refusion étant supérieure à 11 °C, caractérisé en ce que le polyéthylène présente une morphologie cristalline présentant une distribution bimodale des espacements de plis des chaînes moléculaires, l'un des groupes desdits espacements étant de 200 à 800 nm et l'autre de 5 à 50 nm.

2. Le polyéthylène de la revendication 1 sous la forme d'un article dont les dimensions sont d'au moins 2,5 cm sur au moins 2,5 cm.

3. Le polyéthylène de la revendication 1 sous la forme d'un article dont la plus petite dimension est d'au moins 0,5 cm.

4. Un procédé pour la préparation du polyéthylène de la revendication 1 essentiellement constitué par les opérations consistant :
(a) à former un article en un polyéthylène linéaire de poids moléculaire ultra-haut ayant un poids moléculaire d'au moins 400 000 ;
(b) à soumettre ledit article à un fluide sous une pression d'au moins 200 MPa mais non supérieure à 269 MPa et à une température de 190 à 300 °C pendant au moins 0,5 heure ;
(c) à réduire la température à une valeur d'environ 160 à 175°C ou moins tout en maintenant la pression à une valeur au moins égale à 200 MPa mais non supérieure à 260 MPa, la vitesse d'abaissement de la température étant telle qu'il n'apparaisse sensiblement pas de gradients de température dans l'article façonné ; et
(d) à refroidir à une température non supérieure à environ 130 °C et à abaisser la pression à environ 100 kPa de manière à éviter la refusion dudit article.

5. Le procédé de la revendication 4 dans lequel l'opération (a) est effectuée après l'opération (d).

6. Le procédé de la revendication 4 dans lequel ledit fluide est de l'eau.

7. Le procédé de la revendication 4 dans lequel la température de l'opération (b) est de 200 à 260 °C.

8. Le procédé de la revendication 4 dans lequel la température et la pression de l'opération (d) sont maintenues pendant au moins 1 heure.

9. Le procédé de la revendication 4 dans lequel on enlève une mince couche de la surface de l'article après l'opération (d).

10. Le procédé de la revendication 4 dans lequel la vitesse de refroidissement de l'opération (c) est non supérieure à 35 °C par heure.

11. Le procédé de la revendication 10 dans lequel la vitesse de refroidissement de l'opération (c) est non supérieure à 10 °C par heure.
